(19) Europäisches Patentamt
European Patent Office
Office européen des brevets

(11) Numéro de publication : **0 452 168 A1**

(12) **DEMANDE DE BREVET EUROPEEN**

(21) Numéro de dépôt : **91400642.4**

(22) Date de dépôt : **08.03.91**

(51) Int. Cl.$^5$ : **C07C 251/24**, C07C 251/22, C07C 211/54, C07C 211/55, C07C 249/02, C07C 209/52

(30) Priorité : **13.03.90 FR 9003447**

(43) Date de publication de la demande : **16.10.91 Bulletin 91/42**

(84) Etats contractants désignés : **AT BE CH DE DK ES FR GB GR IT LI LU NL SE**

(71) Demandeur : **RHONE-POULENC CHIMIE**
**25, quai Paul Doumer**
**F-92408 Courbevoie Cédex (FR)**

(72) Inventeur : **Desmurs, Jean-Roger**
**La Jonquière - Route de Ternay, Communay**
**F-69360 Saint Symphorien d'Ozon (FR)**
Inventeur : **Kempf, Hubert**
**Résidence Pierrefontaine, 26, rue Daguerre**
**F-68200 Mulhouse (FR)**
Inventeur : **Ratton, Serge**
**13, rue d'Ayen**
**F-78100 Saint Germain en Laye (FR)**
Inventeur : **Stephan, Dominique**
**30, rue Léo Lagrange**
**F-69200 Venissieux (FR)**

(74) Mandataire : **Dutruc-Rosset, Marie-Claude et al**
**RHONE-POULENC CHIMIE Service Brevets Chimie 25, Quai Paul Doumer**
**F-92408 Courbevoie Cédex (FR)**

(54) **Procédé de préparation de N-phényl benzoquinone-imine.**

(57) La présente invention concerne un procédé de préparation de N-phényl benzoquinone-imine par oxydation de la N-(hydroxy-4 phényl)aniline, caractérisé en ce que l'on opère en milieu liquide au moins partiellement organique, en présence d'un composé basique et éventuellement en présence d'une quantité efficace d'au moins un composé métallique du groupe comprenant les composés du manganèse, du cuivre, du cobalt et du nickel.

Elle concerne également la réaction de la N-phényl benzoquinone-imine ainsi obtenue avec une amine aliphatique ou cycloaliphatique, afin de préparer une N-phényl N'-(cyclo)alkyl paraphénylène diamine et une N-phényl N'-(cyclo)alkyl cyclohexadiène-2,5 diimine-1,4.

Ce dernier composé peut être facilement réduit en N-phényl N'-(cyclo)alkyl paraphénylène diamine correspondante.

EP 0 452 168 A1

Jouve, 18, rue Saint-Denis, 75001 PARIS

# PROCEDE DE PREPARATION DE N-PHENYL BENZOQUINONE-IMINE

La présente invention concerne un procédé de préparation de N-phényl benzoquinone-imine par oxydation de la N-(hydroxy-4 phényl)aniline.

Elle concerne également la réaction de la N-phényl benzoquinone-imine ainsi obtenue avec une amine aliphatique ou cycloaliphatique, afin de préparer une N-phényl N'-(cyclo)alkyl paraphénylène diamine et une N-phényl N'-(cyclo)alkyl cyclohexadiène-2,5 diimine-1,4.

Ce dernier composé peut être facilement réduit en N-phényl N'-(cyclo)alkyl paraphénylène diamine correspondante.

Les N-phényl N'-alkyl paraphénylène diamines sont actuellement notamment préparées par réduction, puis alkylation de la paranitrodiphénylamine.

Ainsi le brevet US-A-4 463 191 décrit un procédé de réduction-alkylation en une étape de paranitrodiphénylamine, avec des cétones ou des aldéhydes, en présence d'un complexe palladium/acide anthranilique fixé sur un polymère non réticulé et d'une résine sulfonée. Ce catalyseur nécessite une préparation en plusieurs étapes.

La présente invention permet de préparer la N-phényl benzoquinone-imine, qui peut être en particulier un intermédiaire des N-phényl N'-(cyclo)alkyl paraphénylène diamines.

L'invention est plus précisément un procédé d'oxydation de la N-(hydroxy-4 phényl)aniline en N-phényl benzoquinone-imine, caractérisé en ce que l'on opère en milieu liquide au moins partiellement organique, en présence d'un composé basique.

Le procédé d'oxydation selon l'invention peut être mis en oeuvre en présence d'une quantité efficace d'au moins un composé métallique du groupe comprenant les composés du manganèse, du cuivre, du cobalt et du nickel.

Selon une première variante du procédé de l'invention, le milieu liquide dans lequel est effectuée la réaction d'oxydation est entièrement organique et le composé basique est une amine tertiaire.

Généralement l'amine tertiaire répond à la formule générale (I) :

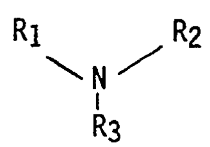

(I)

dans laquelle :

- $R_1$ et $R_2$, identiques ou différents, représentent un radical alkyle, linéaire ou ramifié, ayant 1 à 12 atomes de carbone ou un radical phényle ;
- $R_3$ représente :
  - . un radical alkyle, linéaire ou ramifié, ayant 1 à 12 atomes de carbone,
  - . un radical alcényle, linéaire ou ramifié, ayant 2 à 12 atomes de carbone,
  - . un radical phényle,
  - . un radical phényle comportant un ou deux radicaux alkyles, linéaires ou ramifiés, ayant 1 à 4 atomes de carbone,
  - . un radical cyclohexyle ou cyclopentyle
  - . un radical benzyle ou phénéthyle.

Parmi les amines tertiaires de formule (I) les trialkylamines non quaternisables, dont les symboles $R_1$, $R_2$ et $R_3$ sont des radicaux alkyles, linéaires ou ramifiés, ayant 1 à 12 atomes de carbone, sont particulièrement préférées.

Comme exemples non limitatif d'amines tertiaires de formule (I), on peut citer notamment la diisopropyléthylamine, la dicyclohexyléthylamine, la di(méthyl-1 propyl) éthylamine, la n-propyl diisopropylamine, la diisobutyl éthylamine, l'allyl diisopropylamine, l'allyl diisobutylamine, la diphényl éthylamine.

L'amine tertiaire représente habituellement en moles au moins 1 % de la quantité molaire de N-(hydroxy-4 phényl) aniline introduite.

De préférence elle représente en moles de 1 % à 1000 % de la quantité molaire de N-(hydroxy-4 phényl) aniline et encore plus préférentiellement de 50 % à 300 %.

L'amine tertiaire peut constituer elle-même le solvant organique de la réaction selon le procédé de l'invention.

On peut également conduire la réaction dans un tiers-solvant inerte dans les conditions opératoires, différent de l'amine tertiaire.

Ce tiers-solvant peut être choisi parmi les alcools comme par exemple le méthanol, l'éthanol, l'alcool isopropylique, le trifuoro éthanol, le cyclohexanol ; les éthers comme le diisopropyléther, le méthyl-tertiobutyléther, le tétrahydrofuranne, le dioxanne ; les cétones comme l'acétone, la méthylisobutylcétone, la cyclohexanone ; les nitriles comme l'acétonitrile ; les amides N-alkylés comme le diméthylformamide, le diméthylacétamide, la N-méthylpyrrolidone ; les hydrocarbures aliphatiques ou aliphatiques chlorés comme l'hexane, le dichlorométhane, le dichloroéthane, le trichlorométhane (ou chloroforme) ; les hydrocarbures aromatiques ou aromatiques chlorés comme le toluène, les xylènes, le chlorobenzène, les dichlorobenzènes ; les hydrocarbures cycloaliphatiques comme le cyclohexane ; les esters comme l'acétate d'éthyle ; les sulfoxydes comme le diméthylsulfoxyde.

Parmi ces solvants, les alcools conviennent tout particulièrement bien, notamment le méthanol, l'éthanol, l'isopropanol et le trifluorométhyléthanol.

Selon une deuxième variante de l'invention, le milieu liquide dans lequel est effectué la réaction d'oxydation est hydro-organique et le composé basique est un hydroxyde de métal alcalin.

Le solvant organique qui constitue avec l'eau le milieu liquide est habituellement un solvant non miscible à l'eau choisi parmi les alcools, les éthers, les cétones, les nitriles, les hydrocarbures aliphatiques et aliphatiques chlorés ; les hydrocarbures aromatiques et aromatiques chlorés, les hydrocarbures cycloaliphatiques.

Parmi ces solvants les hydrocarbures aliphatiques ou aliphatiques chlorés comme l'hexane, le dichlorométhane, le chloroforme, le dichloroéthane, le trichloroéthane ; les hydrocarbures aromatiques ou aromatiques chlorés comme le toluène, les xylènes, le chlorobenzène, les dichlorobenzènes ; les hydrocarbures cycloaliphatiques comme le cyclohexane ; sont préférés car ils dissolvent au moins partiellement la N-phényl benzoquinone-imine formée.

Parmi les hydroxydes de métal alcalin, la soude est généralement préférée pour des raisons économiques.

Habituellement la quantité d'hydroxyde de métal alcalin est telle que l'on a un rapport molaire hydroxyde de métal alcalin/N-(hydroxy-4 phényl)aniline de 5 % à 500 % et de préférence de 10 % à 100 %.

Le rapport volumique eau/solvant organique est le plus souvent compris entre 0,01 et 100 et de préférence ente 0,1 et 10.

Les composés du manganèse, du cuivre, du cobalt et du nickel qui peuvent être utilisés dans la présente invention sont généralement choisis parmi les sels de manganèse, de cuivre, de cobalt ou de nickel des acides carboxyliques mono ou polyfonctionnels tels que par exemple des acides acétique, propionique, succinique, benzoïque, des acides halogèno-carboxyliques comme les acides chloroacétiques et fluoroacétiques ; des acides halogèno-sulfoniques tels que l'acide trifluorométhanesulfonique ; des acides minéraux comme les acides halohydriques tels que l'acide chlorhydrique, l'acide bromhydrique, l'acide fluorhydrique ou l'acide iodhydrique ; l'acide sulfurique ; l'acide nitrique ; l'acide perchlorique ; les acides phosphoriques, l'acide carbonique ainsi que les oxydes ou hydroxydes de manganèse, de cuivre, de cobalt ou de nickel.

L'oxydation peut être effectuée par l'oxygène ou un gaz en contenant tel que l'air ou un mélange oxygène-/azote par exemple.

L'oxygène ou le gaz qui en contient peut être mis en oeuvre sous pression atmosphérique, comme par exemple par barbotage dans le mélange réactionnel ou simple circulation dans le réacteur où se passe la réaction d'oxydation, ou sous pression plus élevée.

Généralement la pression partielle de l'oxygène est de 0,02 MPa (0,2 bar) à 2 MPa (20 bars).

Le composé métallique du manganèse, du cuivre, du cobalt ou du nickel joue alors un rôle de catalyseur et il représente habituellement de 0,1 % à 100 % en moles par rapport à la N-(hydroxy-4 phényl)aniline.

De préférence ce rapport molaire composé du manganèse, du cuivre, du cobalt ou du nickel/(N-(hydroxy-4 phényl)aniline est de 2 % à 20 %.

Dans le cadre de la première variante du procédé de l'invention mettant en oeuvre un milieu entièrement organique et une amine tertiaire, le composé du manganèse, du cuivre, du cobalt ou du nickel peut être luimême l'agent oxydant.

Dans ce cas le rapport molaire composé du manganèse, du cuivre, du cobalt ou du nickel/N-(hydroxy-4 phényl)aniline est alors de 0,9 à 3 et de préférence de 1,0 à 1,5.

Dans le cadre de la deuxième variante du procédé de l'invention mettant en oeuvre un milieu hydro-organique et une base minérale, l'agent oxydant peut aussi être un oxydant chimique habituel, tel que par exemple le peroxyde d'hydrogène, en quantité sensiblement au moins stoechiométrique par rapport à la N-(hydroxy-4 phényl)aniline.

La température à laquelle est réalisée le procédé de l'invention peut varier dans de larges limites.

Habituellement on opère entre 5°C et 120°C et de préférence entre 10°C et 80°C.

La N-phényl benzoquinone-imine obtenue selon le présent procédé est un composé intermédiaire qui sert à préparer la N-phényl N'-(cyclo)alkylparaphénylène diamine et la N-phényl N'-(cyclo)alkyl cyclohexadiène-2,5

diimine-1,4 correspondantes, par condensation avec une amine primaire aliphatique ou cycloaliphatique.

Pour cela elle peut être isolée par les méthodes usuelles utilisées en chimie.

Plus précisément la présente invention concerne également un procédé de préparation de N-phényl N'-(cyclo)alkyl paraphénylène diamine de formule générale (II) :

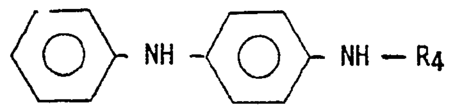

(II)

et de N-phényl N'-(cyclo)alkyl cyclohexadiène-2,5 diimine-1,4 de formule générale (III) :

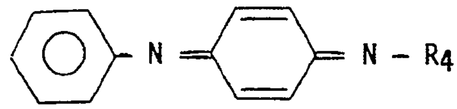

(III)

dans lesquelles $R_4$ représente un radical alkyle, linéaire ou ramifié, ayant 1 à 12 atomes de carbone ou un radical cycloalkyle ayant 5 à 8 atomes de carbone,

caractérisé en ce que l'on fait réagir en milieu liquide la N-phényl benzoquinone-imine avec une amine de formule générale (IV) :

$$R_4 - NH_2 \qquad (IV)$$

dans laquelle $R_4$ a les significations indiquées précédemment.

Comme amines de formule (IV), on peut citer plus particulièrement la diméthyl-1,3 butylamine, l'éthylamine, la méthylamine, l'isopropylamine, la n-propylamine,la méthyl-2 propylamine, la n-butylamine, la méthyl-1 butylamine, la diméthyl-1,2 propylamine, la diméthyl-1,1 propylamine, la méthyl-2 butylamine, la méthyl-3 butylamine, l'éthyl-1 propylamine, la diméthyl-1,1 éthylamine, la pentylamine, l'hexylamine, la méthyl-1 pentylamine, la méthyl-2 pentylamine, la méthyl-3 pentylamine, la méthyl-4 pentylamine, la diméthyl-1,2 butylamine, l'éthyl-1 butylamine, l'éthyl-2 butylamine, l'heptylamine et ses isomères, l'octylamine et ses isomères, la nonilamine et ses isomères, la décylamine et ses isomères, la cyclopentylamine, la cyclohexylamine, les méthylcyclohexylamines et la cyclooctylamine.

La diméthyl-1,3 butylamine est tout particulièrement préférée, car elle conduit à la N-phényl N'-(diméthyl-1,3 butyl) paraphénylène diamine qui est un agent antiozonant très utilisé dans les caoutchoucs naturels ou synthétiques.

Le rapport molaire amine de formule (IV)/N-phényl benzoquinone-imine est généralement compris entre 0,9 et 6.

De préférence ce rapport est compris entre 0,9 et 1,3.

Le liquide dans lequel est conduite la réaction peut être tout solvant, au moins partiel, de la N-phényl benzoquinone -imine et de l'amine de formule (IV), dans la mesure où ledit solvant ne réagit pas avec les réactifs dans les conditions opératoires.

Ainsi on peut utiliser notamment les alcools comme par exemple le méthanol, l'éthanol, l'alcool isopropylique, le trifuoro éthanol, le cyclohexanol ; les éthers comme le diisopropyléther, le méthyl-tertiobutyléther, le tétrahydrofuranne, le dioxanne ; les cétones comme l'acétone, la méthylisobutylcétone, la cyclohexanone ; les nitriles comme l'acétonitrile ; les amides N-alkylés comme le diméthylformamide, le diméthylacétamide, la N-méthylpyrrolidone ; les hydrocarbures aliphatiques ou aliphatiques chlorés comme l'hexane, le dichlorométhane, le dichloroéthane, le trichlorométhane (ou chloroforme) ; les hydrocarbures aromatiques ou aromatiques chlorés comme le toluène, les xylènes, le chlorobenzène, les dichlorobenzènes ; les hydrocarbures cycloaliphatiques comme le cyclohexane ; les esters comme l'acétate d'éthyle ; les sulfoxydes comme le diméthylsulfoxyde.

Parmi ces solvants les alcools sont particulièrement préférés.

La concentration initiale de la N-phényl benzoquinone-imine dans le solvant peut varier dans de larges limites. Elle se situe généralement entre 1 % et 40 % en poids par rapport au poids de mélange réactionnel.

De préférence on utilise des concentrations initiales de N-phényl benzoquinone-imine de 4 % à 25 % en poids par poids de mélange réactionnel.

La température à laquelle est réalisée la réaction entre la N-phényl benzoquinone-imine et l'amine de formule (IV) est généralement de 5°C à 80°C et de préférence de 10°C à 60°C.

La N-phényl N'-(cyclo)alkyl cyclohexadiène-2,5 diimine-1,4 obtenue souvent majoritairement dans la réaction de la N-phényl benzoquinone-imine avec l'amine de formule (IV) peut facilement être réduite en N-phényl

N'-(cyclo)alkyl paraphénylène diamine correspondante par tout moyen connu : hydrogénation par $H_2$ éventuellement en présence d'un catalyseur habituel ; réduction chimique par exemple à l'aide d'un hydrure métallique.

Les N-phényl N'-(cyclo)alkyl paraphénylène diamines sont des composés qui peuvent être utilisés comme antiozonants dans les vulcanisats à base de caoutchouc naturel ou d'élastomères de synthèse.

Les exemples qui suivent illustrent l'invention.

EXEMPLE 1 A 3 ET ESSAIS COMPARATIFS A, B et C

Dans un réacteur en verre de 30 $cm^3$, on charge :

- N-(hydroxy-4 phényl) aniline : 1 g (5,4 mmol)
- sel métallique (indiqué dans le tableau I ci-après) : 5,4 mmol
- méthanol : 5 $cm^3$
- diisopropyléthylamine : 5 mmol ou 0 (essais comparatifs)

Le mélange réactionnel est agité pendant 2 heures à température ambiante (20 à 25°C).

Après filtration du sel métallique, le mélange réactionnel final est dosé par chromatographie liquide haute performance (CLHP).

Le tableau I suivant indique les résultats obtenus.

Les abréviations suivantes sont utilisées :

TT = taux de transformation
RR = rendement obtenu
RT = rendement par rapport au réactif transformé
HPA ≙ N-(hydroxy-4 phényl)aniline
PQI = N-phényl benzoquinone-imine

TABLEAU I

| ESSAIS | Sel métallique | Diisopropyl éthylamine (mmol) | TT % HPA | RR % PQI | RT % PQI |
|---|---|---|---|---|---|
| Exemple 1 | Mn $Cl_2$, $2H_2O$ | 5 | 70 | 10 | 15 |
| Essai A | $MnCl_2$, $2H_2O$ | 0 | 1,5 | 0,3 | 20 |
| Exemple 2 | $CuSO_4$ | 5 | 63 | 25 | 40 |
| Essai B | $CuSO_4$ | 0 | 6 | 3 | 50 |
| Exemple 3 | Cu Cl | 5 | 39 | 27 | 69 |
| Essai C | Cu Cl | 0 | 21 | 7 | 34 |

EXEMPLES 4 A 9 ET ESSAIS D à H

Dans un réacteur en verre de 30 $cm^3$, on charge :

- N-(hydroxy-4 phényl) aniline : 1 g (5,4 mmol)
- sel métallique (nature et quantité indiquées dans le tableau II ci-après)
- méthanol : 5 $cm^3$
- diisopropyléthylamine : quantité indiquée dans le tableau II ci-après

On agite le mélange réactionnel pendant 2 heures à température ambiante (20 à 25°C) tout en introduisant de l'air à l'aide d'un tube capillaire.

Après filtration du sel métallique, le mélange réactionnel final est dosé par CLHP. Les résultats sont rassemblés dans le tableau II.

Les abréviations utilisées sont les mêmes que le tableau I.

TABLEAU II

| Essais | Sel métallique | | Diisopropyl éthylamine (mmol) | TT % HPA | RR % PQI | RT % PQI |
|---|---|---|---|---|---|---|
| | Nature | Quantité (mmol) | | | | |
| Exemple 4 | $CuSO_4$ | 0,54 | 5 | 82 | 56 | 68 |
| Essai D | $CuSO_4$ | 0,54 | 0 | 3 | 1,2 | 40 |
| Exemple 5 | Co $Cl_2$ | 0,54 | 5 | 46 | 29 | 62 |
| Essai E | Co $Cl_2$ | 0,54 | 0 | 2 | 0,4 | 20 |
| Exemple 6 | Co $Cl_2$ | 0,25 | 0,5 | 28 | 25 | 89 |
| Essai F | Co $Cl_2$ | 0,25 | 0 | 12 | 4 | 33 |
| Exemple 7 | $MnCl_2$, $4H_2O$ | 0,25 | 5 | 78 | 70,5 | 90 |
| Essai G | $MnCl_2$, $4H_2O$ | 0,25 | 0 | 1,6 | - | - |
| Exemple 8 | $CuSO_4$, $4H_2O$ | 0,15 | 0,75 | 76 | 55 | 72 |
| Exemple 9 | $CuSO_4$, $4H_2O$ | 0,15 | 7,5 | 91 | 82 | 90 |
| Essai H | $CuSO_4$, $4H_2O$ | 0,15 | 0 | 7 | 4 | 60 |

EXEMPLES 10 A 11

Dans un réacteur en verre de 30 $cm^3$, on charge :

- N-(hydroxy-4 phényl) aniline : 1 g (5,4 mmol)
- Cu $SO_4$ : 0,54 mmol
- solvant (indiqué dans le tableau III): 5 $cm^3$
- diisopropyléthylamine : 5 mmol

On agite le mélange réactionnel pendant 2 heures à température ambiante (20 à 25°C) tout en introduisant de l'air à l'aide d'un tube capillaire.

Après filtration du sel métallique, le mélange réactionnel final est dosé par CLHP. Les résultats sont rassemblés dans le tableau III ci-après.

Les abréviations utilisées sont les mêmes que le tableau I.

TABLEAU III

| ESSAIS | SOLVANT | TT % HPA | RR % PQI | RT % PQI |
|---|---|---|---|---|
| Exemple 10 | Chloroforme | 5 | 5 | 100 |
| Exemple 11 | Trifluoréthanol | 21 | 19 | 90 |

EXEMPLE 12

Dans un réacteur en verre de 30 $cm^3$, on charge :

- Cu Cl : 1,07 g (10,8 mmol)
- diisopropyléthylamine : 5 $cm^3$

On charge par ajouts successifs 1 g (5,4 mmol) de N-(hydroxy-4 phényl)aniline.
Le mélange réactionnel est agité 2 heures à température ambiante (20 à 25°C).
Après filtration du sel métallique, on dose le mélange réactionnel final par CLHP.
On obtient les résultats suivants :

TT de HPA = 44 %
RR en PQI = 42 %
RT en PQI = 96 %

EXEMPLES 13 A 15

Dans un réacteur tubulaire muni à la partie inférieure d'un verre fritté, on charge :

- N-(hydroxy-4 phényl)aniline : 1 g (5 mmol)
- toluène : 25 $cm^3$
- solution aqueuse de soude 2 N (quantité indiquée dans le tableau IV)

TABLEAU IV

| ESSAIS | NaOH (mmol) | TT % HPA | RR % PQI | RT % PQI |
|---|---|---|---|---|
| Exemple 13 | 25 | 100 | 41 | 41 |
| Exemple 14 | 5 | 99 | 51 | 52 |
| Exemple 15 | 0,5 | 21 | 17 | 81 |

EXEMPLES 16 A 21

Dans un réacteur en verre de 30 $cm^3$, on charge:

- N-phényl benzoquinone-imine : 1,82 g (10 mmol)
- diméthyl-1,3 butylamine : 1 g (10 mmol)
- solvant (indiqué dans le tableau V) : 10 $cm^3$

Le mélange réactionnel est agité 24 heures à 25°C.

Le mélange réactionnel final est dosé par CLHP. On obtient les résultats suivants.

Les abréviations utilisées sont les mêmes que le tableau I ainsi que les abréviations suivantes supplémentaires.

– PDBCD = N-phényl N'-(diméthyl-1,3 butyl) cyclohexadiène-2,5 diimine-1,4

– PMBPD = N-phényl N'-(diméthyl-1,3 butyl) paraphénylène diamine

TABLEAU V

| Essais | Solvant | TT % PQI | RR % PDBCD | RR % PMBPD | RT % PDBCD | RT % PMBPD |
|---|---|---|---|---|---|---|
| Exemple 16 | Ethanol | 83 | 59 | 19 | 63 | 23 |
| Exemple 17 | Propanol-2 | 65 | 44 | 22 | 68 | 34 |
| Exemple 18 | Dichlorométhane | 22 | 1 | 12 | 4,5 | 54 |
| Exemple 19 | Ether isopropylique | 1 | 0,5 | 0,5 | 50 | 50 |
| Exemple 20 | Toluène | 59 | 9 | 14 | 15 | 24 |
| Exemple 21 | o.Dichlorobenzène | 45 | 1 | 23 | 2,2 | 51 |

EXEMPLES 22 A 24

Dans un réacteur de 200 $cm^3$, on charge :

- N-phényl benzoquinone-imine : 4,57 g (25 mmol)
- diméthyl-1,3 butylamine : 2,52 g (25 mmol)
- méthanol : 100 $cm^3$

Le mélange réactionnel est agité pendant 24 heures à la température indiquée dans le tableau VI.

On dose par CLHP le mélange réactionnel final.

On obtient les résultats suivants.

TABLEAU VI

| Essais | Température (°C) | TT % PQI | RR % PDBCD | RR % PMBPD | RT % PDBCD | RT % PMBPD |
|---|---|---|---|---|---|---|
| Exemple 22 | 25 | 91 | 76,5 | 8 | 84 | 9 |
| Exemple 23 | 50 | 96 | 86 | 10,5 | 90 | 11 |
| Exemple 24 | 65 | 96 | 66 | 26 | 69 | 27 |

EXEMPLES 25 A 27

Dans un réacteur de 30 $cm^3$, on charge :

- N-phényl benzoquinone-imine : 0,91 g (5 mmol)
- diméthyl-1,3 butylamine : 5 ou 25 mmol (voir tableau VII)
- solvant (indiqué dans le tableau VII) : 5 $cm^3$

Le mélange réactionnel est agité pendant 20 heures à 25°C.

On dose par CLHP le mélange réactionnel final.

On obtient les résultats suivants.

TABLEAU VII

| Essais | Solvant | Diméthyl-1,3 butylamine (mmol) | TT % PQI | RR % PDBCD | RR % PMBPD | RT % PDBCD | RT % PMBPD |
|---|---|---|---|---|---|---|---|
| Exemple 25 | Méthanol | 5 | 99 | 78 | 21 | 79 | 21 |
| Exemple 26 | Méthanol | 25 | 99 | 17 | 49 | 17 | 49,5 |
| Exemple 27 | Chloroforme | 5 | 57 | 21 | 14 | 37 | 24,5 |

**Revendications**

**1** - Procédé d'oxydation de la N-(hydroxy-4 phényl)aniline en N-phényl benzoquinone-imine, caractérisé en ce que l'on opère en milieu liquide au moins partiellement organique, en présence d'un composé basique.

**2** - Procédé selon la revendication 1, caractérisé en ce que l'on opère en présence d'une quantité efficace d'au moins un composé métallique du groupe comprenant les composés du manganèse, du cuivre, du cobalt et du nickel.

**3** - Procédé selon l'une des revendications 1 ou 2, caractérisé en ce que le milieu liquide est entièrement organique et le composé basique est une amine tertiaire représentant au moins 1 % en moles de la quantité molaire de N-(hydroxy-4 phényl)aniline.

**4** - Procédé selon la revendication 3, caractérisé en ce que l'amine tertiaire répond à la formule générale (I) :

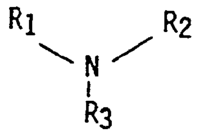

(I)

dans laquelle :

– $R_1$ et $R_2$, identiques ou différents, représentent un radical alkyle, linéaire ou ramifié, ayant 1 à 12 atomes de carbone ou 1 radical phényle ;

– $R_3$ représente :

. un radical alkyle, linéaire ou ramifié, ayant 1 à 12 atomes de carbone,

. un radical alcényle, linéaire ou ramifié, ayant 2 à 12 atomes de carbone,

. un radical phényle,

. un radical phényle comportant 1 ou 2 radicaux alkyles, linéaires ou ramifiés, ayant 1 à 4 atomes de

carbone,
. un radical cyclohexyle.

**5** - Procédé selon l'une des revendications 3 ou 4, caractérisé en ce que l'amine tertiaire représente en moles de 1 % à 1000 % de la quantité molaire de N-(hydroxy-4 phényl)aniline et de préférence de 50 % à 300 %.

**6** - Procédé selon l'une des revendications 3 à 5, caractérisé en ce que l'amine tertiaire est une trialkylamine non quaternisable, de formule (I) dont les symboles $R_1$, $R_2$ et $R_3$ sont des radicaux alkyles, linéaires ou ramifiés, ayant 1 à 12 atomes de carbone.

**7** - Procédé selon l'une des revendications 3 à 6, caractérisé en ce que l'amine tertiaire constitue le solvant de la réaction.

**8** - Procédé selon l'une quelconque des revendications 3 à 6. caractérisé en ce que la réaction est conduite dans un solvant inerte choisi parmi les alcools, les éthers, les cétones, les nitriles, les amides N-alkylés, les hydrocarbures aliphatiques ou aliphatiques chlorés, les hydrocarbures aromatiques ou aromatiques chlorés, les hydrocarbures cycloaliphatiques, les esters, les sulfoxydes.

**9** - Procédé selon l'une des revendications 1 ou 2, caractérisé en ce que le milieu de la réaction est un milieu biphasique hydro-organique et le composé basique est un hydroxyde de métal alcalin.

**10** - Procédé selon la revendication 9, caractérisé en ce que la quantité d'hydroxyde de métal alcalin est telle que l'on a un rapport molaire hydroxyde de métal alcalin/N-(hydroxy-4 phényl)aniline de 5 % à 500 % et de préférence de 10 % à 100 %.

**11** - Procédé selon la revendication 9, caractérisé en ce que le solvant organique constituant l'une des phases est choisi parmi les hydrocarbures aliphatiques, les hydrocarbures aliphatiques chlorés, les hydrocarbures aromatiques, les hydrocarbures aromatiques chlorés les hydrocarbures cycloaliphatiques, dans lesquels la N-phényl benzoquinone-imine est au moins partiellement soluble.

**12** - Procédé selon l'une des revendications 9 à 11, caractérisé en ce que le composé basique est la soude et le rapport volumique eau/solvant organique est compris entre 0,01 et 100 et de préférence entre 0,1 et 10.

**13**- Procédé selon l'une des revendications 1 à 12, caractérisé en ce que l'oxydation est effectuée par l'oxygène a un gaz en contenant et en présence de 0,1 % à 100 % en moles de composé du manganèse, du cuivre, du cobalt ou du nickel par rapport à la N-(hydroxy-4 phényl) aniline et de préférence de 2 % à 20 % en moles.

**14** - Procédé selon l'une des revendications 1 à 8, caractérisé en ce que l'oxydation est effectuée par un composé du manganèse, du cuivre, du cobalt ou du nickel avec un rapport molaire composé métallique/N-(hydroxy-4 phényl)aniline de 0,9 à 3,0 et de préférence de 1,0 à 1,5.

**15** - Procédé selon l'une des revendications 1 à 14, caractérisé en ce que les composés métalliques sont choisis parmi les sels de manganèse, de cuivre, de cobalt ou de nickel des acides carboxyliques mono ou polyfonctionnels, des acides halogénocarboxyliques ; les sels de manganèse, de cuivre, de cobalt ou de nickel des acides minéraux tels que les acides halohydriques, l'acide sulfurique, l'acide nitrique, l'acide perchlorique, les acides phosphoriques, l'acide carbonique ; les oxydes ou hydroxydes de manganèse, de cuivre, de cobalt ou de nickel.

**16** - Procédé selon l'une des revendications 1 à 15, caractérisé en ce que l'on opère à une température de 5°C à 120°C et de préférence de 10°C à 80°C.

**17** - Procédé de préparation de N-phényl N'-(cyclo) alkyl paraphénylène diamine de formule générale (II) :

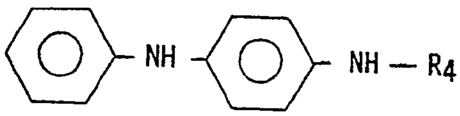

(II)

et de N-phényl N'-(cyclo)alkyl cyclohexadiène-2,5 diimine-1,4 de formule générale (III) :

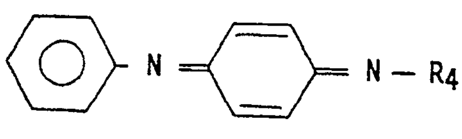

(III)

dans lesquelles $R_4$ représente un radical alkyle, linéaire ou ramifié, ayant 1 à 12 atomes de carbone ou un radical cycloalkyle ayant 5 à 8 atomes de carbone,
caractérisé en ce que l'on fait réagir en milieu liquide la N-phényl benzoquinone-imine, obtenue par le procédé selon l'une des revendications 1 à 16, avec une amine de formule générale (IV) :

$$R_4 - NH_2 \qquad (IV)$$

dans laquelle $R_4$ a les significations indiquées précédemment.

**18** - Procédé selon la revendication 17, caractérisé en ce que l'amine de formule (IV) est choisi parmi la diméthyl-1,3 butylamine, l'éthylamine, la méthylamine, l'isopropylamine, la n-propylamine,la méthyl-2 propylamine, la n-butylamine, la méthyl-1 butylamine, la diméthyl-1,2 propylamine, la diméthyl-1,1 propylamine, la méthyl-2 butylamine, la méthyl-3 butylamine, l'éthyl-1 propylamine, la diméthyl-1,1 éthylamine, la pentylamine, l'hexylamine, la méthyl-1 pentylamine, la méthyl-2 pentylamine, la méthyl-3 pentylamine, la méthyl-4 pentylamine, la diméthyl-1,2 butylamine, l'éthyl-1 butylamine, l'éthyl-2 butylamine, l'heptylamine et ses isomères, l'octylamine et ses isomères, la nonilamine et ses isomères, la décylamine et ses isomères, la cyclopentylamine, la cyclohexylamine, les méthylcyclohexylamines et la cyclooctylamine.

**19** - Procédé selon l'une des revendications 17 ou 18, caractérisé en ce que le rapport molaire amine de formule (IV)/N-phényl benzoquinone-imine est compris entre 0,9 et 6 et de préférence entre 0,9 et 1,3.

**20** - Procédé selon l'une des revendications 17 à 19, caractérisé en ce que le liquide dans lequel est conduite la réaction peut être tout solvant, au moins partiel, de la N-phényl benzoquinone-imine et de l'amine de formule (IV), tel que les alcools, les éthers, les cétones, les nitriles, les amides N-alkylés, les hydrocarbures aliphatiques ou aliphatiques chlorés, les hydrocarbures aromatiques ou aromatiques chlorés, les hydrocarbures cycloaliphatiques, les esters, les sulfoxydes.

**21** - Procédé selon l'une des revendications 17 à 20, caractérisé en ce que la concentration initiale de la N-phényl benzoquinone-imine dans le solvant se situe entre 1 % et 40 % en poids par rapport au poids de mélange réactionnel et de préférence entre 4 % et 25 % en poids par poids de mélange réactionnel.

**22** - Procédé selon l'une des revendications 17 à 21, caractérisé en ce que la température à laquelle est réalisée la réaction entre la N-phényl benzoquinone-imine et l'amine de formule (IV) est de 5°C à 80°C et de préférence de 10°C à 60°C.

**23** - Procédé de préparation de N-phényl N'-(cyclo) alkyl paraphénylène diamine de formule générale (II) caractérisé en ce que l'on réduit la N-phényl N'-(cyclo)alkyl cyclohexadiène-2,5 diimine-1,4 préparée selon l'une des revendications 17 à 22 par tout moyen connu.

Office européen des brevets

# RAPPORT DE RECHERCHE EUROPEENNE

Numero de la demande

EP 91 40 0642

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | BE-A- 752 381 (L'OREAL)<br>* revendications 2,4 * | 1 | C 07 C 251/24<br>C 07 C 251/22<br>C 07 C 211/54<br>C 07 C 211/55<br>C 07 C 249/02<br>C 07 C 209/52 |
| A | DE-A-2 331 878 (SNAM PROGETTI)<br>* revendication 1 * | 23 | |
| A | FR-A-2 121 101 (L'OREAL)<br>* revendications 3,6 * | 1,17 | |
| A | RESEARCH DISCLOSURE no. 173, septembre 1978, page 22; 17332 "Process of preparing polyaniline amines" | 1,17 | |

DOMAINES TECHNIQUES RECHERCHES (Int. Cl.5)

C 07 C 251/24
C 07 C 251/22
C 07 C 211/54
C 07 C 211/55
C 07 C 249/02
C 07 C 209/52

Le présent rapport a été établi pour toutes les revendications

| Lieu de la recherche | Date d'achèvement de la recherche | Examinateur |
|---|---|---|
| BERLIN | 19-06-1991 | KAPTEYN H G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)